# EUROPEAN PATENT APPLICATION

(11) **EP 4 321 149 A1**
(43) Date of publication of application: **14.02.2024**
(21) Application number: 22784594.8
(22) Date of filing: 30.03.2022
(51) Int. Cl.: A61K 8/365, A61K 8/362, A61K 8/39, A61K 8/46, A61Q 5/04

(54) **HAIR COSMETIC**

(30) Priority: 09.04.2021 JP 2021066588
(71) Applicant: Kao Corporation, Chuo-ku Tokyo 103-8210 (JP)
(72) Inventor: KUME, Nozomi, Tokyo 131-8501 (JP); TANJI, Noriyuki, Tokyo 131-8501 (JP)
(74) Representative: Hoffmann Eitle
(86) International application number: PCT/JP2022/015782
(87) International publication number: WO 2022/215610

(57) **Abstract**

A hair cosmetic containing the following components (A) to (C):
(A) one or more carboxylic acids selected from the group consisting of a hydroxycarboxylic acid and a polyvalent carboxylic acid having no hydroxy group, or salts thereof;
(B) an aromatic sulfonic acid or a salt thereof; and
(C) a diethylene glycol monoalkyl ether,
in which, a content of the component (A) is 0.8% by mass or more and 10.0% by mass or less.

## Description

### Field of the Invention

The present invention relates to a hair cosmetic.

### Background of the Invention

Among the hair cosmetics, there are the types that are used for the purpose of relaxing or straightening curls of the hair, giving bounce and resilience to the hair, and the like.

For example, in JPH8-92043A (PTL 1), it is described that a hair treatment composition containing a specific organic acid and a specific sulfonic acid is excellent in straightening effect of the curly hair.

In addition, in JPH8-198732A (PTL 2), it is described that by a hair treatment agent composition containing an organic solvent, naphthalenesulfonic acid, and the like, the sufficient bounce/resilience can be imparted to the hair in a short period of treatment, and the effect can be maintained.

Further, in JP2017-119689A (PTL 3), it is described that by a hair treatment method containing three steps of a treatment step with carboxylic acid, a leaving step, and a treatment step with an aromatic sulfonic acid, the fizzy hair can be relaxed, the phase of the fizzy hair is aligned, and the coherence can be improved.

### Summary of the Invention

The present invention relates to a hair cosmetic containing the following components (A) to (C):
(A) one or more carboxylic acids selected from the group consisting of a hydroxycarboxylic acid and a polyvalent carboxylic acid having no hydroxy group, or salts thereof;
(B) an aromatic sulfonic acid or a salt thereof; and
(C) a diethylene glycol monoalkyl ether,
in which, a content of the component (A) is 0.8% by mass or more and 10.0% by mass or less.

### Detailed Description of the Invention

People having the strong fizzy hair, such as African Americans and Caucasians, have additional problems in styling their hair. When drying the hair after washing, the shrinking of the length of the hair occurs while the width of the entire hair expands, which is what is called a shrinkage. In addition, proper elasticity of the hair is also impaired, and it is often very difficult to arrange an ideal hairstyle. Therefore, they have troubles in requiring a large amount of hair cosmetics, times, efforts, and the like for hair styling.

By the techniques described in the above PTL 1 to 3, the effect of inhibiting the shrinkage of the entire hair and imparting an appropriate elastic texture, could not sufficiently be obtained,

In addition, the hair treatment method described in Patent literature 3 also had the problem of requiring at least three steps of the treatment, which is complicated and time-consuming.

The present invention relates to a hair cosmetic that inhibits the shrinkage of the hair during drying after washing, provides an appropriate elastic texture, and is particularly suitable for efficient styling of the strong fizzy hair.

The present invention is based on the finding that a hair cosmetic containing a specific amount of a specific carboxylic acid or a salt thereof, an aromatic sulfonic acid or a salt thereof, and a diethylene glycol monoalkyl ether, is effective in inhibiting the shrinkage of the hair and imparting the appropriate elastic texture.

Specifically, the present invention relates to the following [1] and [2].
[1] A hair cosmetic containing the following components (A) to (C):
   (A) one or more carboxylic acids selected from the group consisting of a hydroxycarboxylic acid and a polyvalent carboxylic acid having no hydroxy group, or salts thereof;
   (B) an aromatic sulfonic acid or a salt thereof; and
   (C) a diethylene glycol monoalkyl ether,
   in which, a content of the component (A) is 0.8% by mass or more and 10.0% by mass or less.
[2] A hair treatment method containing: a step of applying the hair cosmetic according to the item [1] to the hair; and a step of washing the hair, in which the hair cosmetic is applied.

The hair cosmetic of the present invention can inhibit the shrinkage of the hair during drying after washing, provide an appropriate elastic texture, and can be suitably applied to the efficient styling of the strong fizzy hair.

### Brief Description of the Drawing

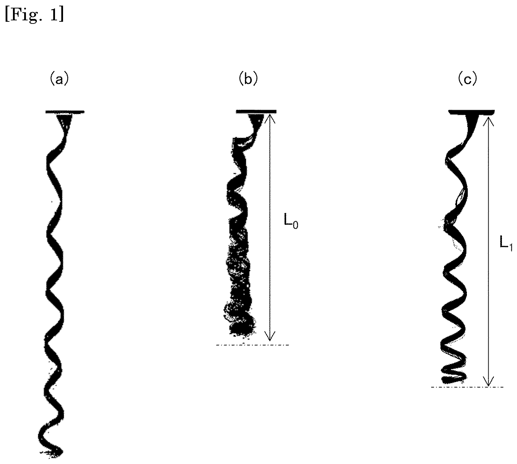

Fig. 1 is a diagram for explaining the shrinkage inhibition evaluation of the hair bundle for the evaluation in the Example, and illustrates a photograph of the state in which the upper end of the hair bundle is fixed and hung, viewed from the front. Respectively, (a) shows the untreated hair bundle for the evaluation in a wet state, (b) shows the untreated hair bundle for the evaluation after 90 minutes passed from the start of naturally drying, and (c) shows the treated hair bundle for the evaluation after 90 minutes passed from the start of a natural drying.

### [Hair cosmetics]

The hair cosmetic of the present invention contains the following components (A) to (C):
(A) one or more carboxylic acids selected from the group consisting of a hydroxycarboxylic acid and a polyvalent carboxylic acid having no hydroxy group, or salts thereof
(B) an aromatic sulfonic acid or a salt thereof; and
(C) a diethylene glycol monoalkyl ether,
in which, a content of the component (A) is 0.8% by mass or more and 10.0% by mass or less.

The hair cosmetic of the present invention can inhibit the shrinkage of the hair during drying after washing, provide the appropriate elastic texture. Therefore, for the strong fizzy hair particularly, it can be reduced the time and the effort required in hairstyling, and is suitable to be applicated.

As an embodiment of the hair cosmetic of the present invention, hair cleansers such as a hair shampoo, a hair rinse, a hair conditioner, a hair treatment, a hair pack, a hair styling agent, and the like are given. Among these, from the viewpoint of further exhibiting the effect of the present invention, the one that is used after washing the hair is preferable, and an embodiment that is used by rinsing after being applied to the hair is more preferable. From this viewpoint, the hair cosmetic of the present invention is preferably a hair rinse, a hair conditioner, a hair treatment, a hair pack or a hair styling agent.

A formulation of the hair cosmetic composition of the present invention may be any formulation, for example, such as liquid, foam, paste or cream. Among these, liquid, paste or cream is preferable, and liquid is more preferable, from the viewpoint of ease of use.

Although the action mechanism of the hair cosmetic of the present invention is not clear, it is presumed as follows.

It is considered that by combining the three components of the component (A), the component (B) and the component (C), a function of the hair to be put together while forming a bundle is enhanced during the period from washing of the hair to drying, the hair is set after the drying is finished in a state in which the shrinkage does not occur often (what is called water setting), and the excellent effect of inhibiting the shrinkage is exhibited.

In addition, since the component (B) has the effect of imparting the elasticity to the hair, and the component (A) and the component (C) have the effect of imparting flexibility to the hair, it is considered that by combining the three components, the hair can be easily adjusted to have the appropriate elasticity.

Therefore, the hair cosmetic of the present invention can be suitably used as a shrinkage inhibitor.

Note that, in the present invention, "to contain a component X" shall be regarded as having the same meaning as "to be formed by mixing a component X".

### <Component (A)>

The hair cosmetic of the present invention contains, as the component (A), one or more carboxylic acids selected from the group consisting of a hydroxycarboxylic acid and a polyvalent carboxylic acid having no hydroxy groups, or salts thereof. As the component (A), one selected from these may be used alone, or two or more thereof may be used in combination.

The carboxylic acids are one or more selected from the group consisting of a hydroxycarboxylic acid and a polyvalent carboxylic acid having no hydroxy group.

As the hydroxycarboxylic acid, from the viewpoint of obtaining the excellent effect of inhibiting the shrinkage and the appropriate elasticity, it is preferably one or more selected from hydroxymonocarboxylic acid having 2 to 5 carbon atoms, hydroxydicarboxylic acid having 3 to 8 carbon atoms, and hydroxytricarboxylic acid having 3 to 8 carbon atoms, more preferably one or more selected from hydroxydicarboxylic acid having 3 to 8 carbon atoms and hydroxytricarboxylic acid having 3 to 8 carbon atoms. As the specific examples of the hydroxycarboxylic acid, glycolic acid, hydroxy acrylic acid, oxybutyric acid, glyceric acid, malic acid, tartaric acid, citric acid, lactic acid, salicylic acid and the like, are given.

As the polyvalent carboxylic acid having no hydroxy group, from the viewpoint of obtaining the excellent effect of inhibiting the shrinkage and the appropriate elasticity, dicarboxylic acid having 3 to 8 carbon atoms and having no hydroxy group is preferable. As the specific examples of the polyvalent carboxylic acid having no hydroxy group, malonic acid, succinic acid, glutaric acid, adipic acid, maleic acid, fumaric acid, phthalic acid, and the like are given.

As the salts of these carboxylic acids, alkali metal salts of carboxylic acid such as sodium salts and potassium salts; alkaline earth metal salts of carboxylic acid such as calcium salts; ammonium salts of carboxylic acid and the like, are given.

Among these, from the viewpoint of the effects of the present invention, the ease of handling, and the like, as the carboxylic acid in the component (A) of the hair cosmetic of the present invention, one or more selected from malic acid, citric acid, succinic acid and lactic acid is preferable, one or more selected from malic acid and citric acid is more preferable, and malic acid is still more preferable. Therefore, as the component (A), one or more selected from malic acid, citric acid, succinic acid, and lactic acid, and the salts thereof are preferable; one or more selected from malic acid, citric acid, and the salts thereof, are more preferable; and malic acid or the salt thereof is still more preferable. As the preferable specific examples of the component (A), malic acid, citric acid, succinic acid, lactic acid, and the like are given. Among these, one or more selected from malic acid and citric acid is more preferable, and malic acid is still more preferable.

In addition, particularly when the component (A) contains one or more carboxylic acids selected from the hydroxydicarboxylic acid having 3 to 8 carbon atoms and the hydroxytricarboxylic acids having 3 to 8 carbon atoms, or the salts thereof, from the viewpoint of obtaining the excellent effect of inhibiting the shrinkage and the appropriate elasticity, the content of the one or more selected from hydroxydicarboxylic acid having 3 to 8 carbon atoms and hydroxytricarboxylic acid having 3 to 8 carbon atoms, or the salts thereof in the component (A) of the hair cosmetic of the present invention, is preferably 50% by mass or more, more preferably 70% by mass or more, still more preferably 90% by mass or more.

Further, in particular, when the component (A) contains one or more carboxylic acids selected from malic acid and citric acid or the salts thereof, from the viewpoint of obtaining the excellent effect of inhibiting the shrinkage and the appropriate elasticity, the content of the one or more carboxylic acids selected from malic acid and citric acid or the salts thereof in the component (A) of the hair cosmetic of the present invention, is preferably 50% by mass or more, more preferably 70% by mass or more, still more preferably 90% by mass or more.

### <Component (B)>

The hair cosmetic of the present invention contains at least an aromatic sulfonic acid or a salt thereof as the component (B), and one selected from these may be used alone, or two or more thereof may be used in combination.

As the aromatic sulfonic acid, naphthalenesulfonic acids, benzenesulfonic acids, azulene sulfonic acids, and the like are given.

As the specific examples of the naphthalenesulfonic acids, 1-naphthalenesulfonic acid, 2-naphthalenesulfonic acid, 2,7-naphthalenedisulfonic acid, 1,5-naphthalenedisulfonic acid, 2,6-naphthalenedisulfonic acid, 1,3,6 -naphthalenetrisulfonic acid, 3,6-dihydroxynaphthalene-2,7-disulfonic acid, 6-methyl-2-naphthalenesulfonic acid, 4-ethyl-1-naphthalenesulfonic acid, 5-isopropyl-1-naphthalenesulfonic acid, 5-butyl-2-naphthalenesulfonic acid, and the like are given.

As the specific examples of the benzenesulfonic acids, benzenesulfonic acid, p-ethylbenzenesulfonic acid, 2,4,6-trimethylbenzenesulfonic acid, xylenesulfonic acid, o-toluenesulfonic acid, p-toluenesulfonic acid, p-cumenesulfone acids, and the like are given.

As the specific examples of the azulene sulfonic acids, 1-azulene sulfonic acid, guaiazulene sulfonic acid, 3-methyl-7-isopropyl-1-azulene sulfonic acid, 7-isopropyl-1-azulene sulfonic acid, 3-phenyl-6-isopropyl -1-azulene sulfonic acid, 1,4-dimethyl-7-isopropyl-2-azulene sulfonic acid, 1,3-azulene disulfonic acid, 4,6,8-trimethyl-1,3-azulene disulfonic acid, 1,3 -bis(1,1-dimethylethyl)-5,7-azulenedisulfonic acid, 1,3-bis(1,1-dimethylethyl)-5-azulenesulfonic acid, and the like are given.

As the salts of these aromatic sulfonic acids, alkali metal salts of aromatic sulfonic acid such as sodium salts and potassium salts; alkaline earth metal salts of aromatic sulfonic acid such as calcium salts; and ammonium salts of aromatic sulfonic acid, and the like are given.

Among these, as the aromatic sulfonic acid of the component (B) of the hair cosmetic of the present invention, from the viewpoint of the effects of the present invention and the ease of handling, one or more selected from naphthalenesulfonic acids and benzenesulfonic acids is preferable, one or more selected from naphthalenesulfonic acid, xylenesulfonic acid, toluenesulfonic acid and cumenesulfonic acid is more preferable, one or more selected from naphthalenesulfonic acid and xylenesulfonic acid is still more preferable, and naphthalenesulfonic acid is even still more preferable. Therefore, the component (B) is preferably one or more selected from naphthalenesulfonic acids, benzenesulfonic acids, and the salts thereof, more preferably, one or more selected from naphthalenesulfonic acid, xylenesulfonic acid, toluenesulfonic acid, cumenesulfonic acid, and the salts thereof, still more preferably, one or more selected from naphthalenesulfonic acid, xylenesulfonic acid and the salts thereof, even still more preferably, one or more selected from naphthalenesulfonic acid and the salt thereof. As the preferred specific examples of the component (B), sodium 2-naphthalenesulfonate, sodium xylenesulfonate, sodium p-toluenesulfonate, and sodium p-cumenesulfonate are given. Among these, sodium 2-naphthalenesulfonate, sodium xylenesulfonate and sodium p-toluenesulfonate are more preferable, sodium 2-naphthalenesulfonate and sodium xylenesulfonate are more preferable, and sodium 2-naphthalenesulfonate is still more preferable.

### <Component (C)>

The hair cosmetic of the present invention contains a diethylene glycol monoalkyl ether as the component (C), and one selected from these may be used alone, or two or more thereof may be used in combination.

The terminal alkyl group of the diethylene glycol monoalkyl ether is, from the viewpoint of the effect of the present invention and hydrophilicity and the like of the component (C), preferably an alkyl group having 2 to 6 carbon atoms, more preferably alkyl group having 2 to 4 carbon atoms. Specifically, one or more selected from diethylene glycol monoethyl ether and diethylene glycol monobutyl ether is preferable, and diethylene glycol monoethyl ether is more preferable.

### <Content of each component>

The content of the component (A) in the hair cosmetic of the present invention is, from the viewpoint of inhibiting the shrinkage of the hair while imparting texture with appropriate elastic texture to the hair, 0.8% by mass or more, preferably 1.0% by mass or more, more preferably 2.5% by mass or more; and is 10.0% by mass or less, preferably 8.0% by mass or less, more preferably 7.0% by mass or less. Taking these viewpoints together, the content of the component (A) in the hair cosmetic is 0.8% by mass or more and 10.0% by mass or less, preferably 1.0% by mass or more and 8.0% by mass or less, more preferably 2.5% by mass or more and 6.0% by mass or less.

Note that when the component (A) is a salt, the content of the component (A) in the hair cosmetic is expressed in terms of the acid equivalent.

The content of the component (B) in the hair cosmetic of the present invention is, from the viewpoint of imparting the appropriate elastic texture to the hair, preferably 0.5% by mass or more, more preferably 1.0% by mass or more, still more preferably 1.5% by mass or more; and is preferably 10.0% by mass or less, more preferably 8.0% by mass or less, still more preferably 6.0% by mass or less. Taking these viewpoints together, the content of the component (B) in the hair cosmetic is preferably 0.5% by mass or more and 10.0% by mass or less, more preferably 1.0% by mass or more and 8.0% by mass or less, still more preferably 1.5% by mass or more and 6.0% by mass or less.

Note that when the component (B) is a salt, the content of the component (B) in the hair cosmetic is expressed in terms of the acid equivalent.

The content of the component (C) in the hair cosmetic of the present invention is, from the viewpoint of inhibiting the shrinkage of the hair, preferably 0.1% by mass or more, more preferably 0.2% by mass or more, still more preferably 0.5% by mass or more; and is preferably 25.0% by mass or less, more preferably 20.0% by mass or less, still more preferably 15.0% by mass or less. Taking these viewpoints together, the content of the component (C) in the hair cosmetic is preferably 0.1% by mass or more and 25.0% by mass or less, more preferably 0.2% by mass or more and 20.0% by mass or less, still more preferably 0.5% by mass or more and 15.0% by mass or less.

In the hair cosmetic of the present invention, from the viewpoint of combining the effects of both inhibiting the shrinkage of the hair and imparting the appropriate elastic texture, the mass ratio [(B)/{(A)+(C)}] of the content of the component (B) with respect to the total content of the component (A) and the component(C) is preferably 0.10 or more, more preferably 0.15 or more, still more preferably 0.18 or more; preferably 3.00 or less, more preferably 2.00 or less, still more preferably 1.00 or less. The above mass ratio [(B)/{(A)+(C)}] is preferably 0.10 or more and 3.00 or less, more preferably 0.15 or more and 2.00 or less, still more preferably 0.18 or more and 1.00 or less.

In the hair cosmetic of the present invention, from the viewpoint of combining the effects of both inhibiting the shrinkage of the hair and imparting the appropriate elastic texture, the mass ratio [(A)/{(A)+(B)}] of the content of the component (A) with respect to the total content of the component (A) and the component(B) is preferably 0.25 or more, more preferably 0.28 or more, still more preferably 0.30 or more; preferably 0.85 or less, more preferably 0.80 or less, till more preferably 0.75 or less. The above mass ratio [(A)/{(A)+(B)}] is preferably 0.25 or more and 0.85 or less, more preferably 0.28 or more and 0.80 or less, still more preferably 0.30 or more and 0.75 or less.

The total content of the components (A) to (C) in the hair cosmetic of the present invention is, from the viewpoint of fully exhibiting the effect of the present invention, preferably 7.5 % by mass or more, more preferably 8.0 % by mass or more, still more preferably 8.5 % by mass or more; preferably 30.0 % by mass or less, more preferably 25.0 % by mass or less, still more preferably 20.0 % by mass or less. Taking these viewpoints together, the total content of the components (A) to (C) in the hair cosmetic is preferably 7.5 % by mass or more and 30.0 % by mass or less, more preferably 8.0 % by mass or more and 25.0 % by mass or less, still more preferably 8.5 % by mass or more and 20.0 % by mass or less.

### <Other components>

The hair cosmetic of the present invention may contain an aqueous medium from the viewpoint of handling and facilitating to exhibit the effects of action of the components (A) to (C), depending on its form and formulation, and the like.

As the aqueous medium, for example, low alcohols such as water, ethanol, and isopropyl alcohol; low molecular diols and triols having 6 or less of carbon atoms such as 1,3-butylene glycol, glycerin, ethylene glycol, propylene glycol, and dipropylene glycol; and the like are given. One of these may be used alone or in combination with two or more of them.

The hair cosmetic of the present invention may also contain further other components such as, for example, pH adjusters, aromatic alcohols, surfactants, antioxidants, oils, vitamins, disinfectants, anti-inflammatory agents, anti-dandruff agents, preservatives, chelating agents, moisturising agents, pearling agents, ceramides, perfumes and UV absorbers.

### <Production method>

The hair cosmetic of the present invention may be manufactured by the usual methods. For example, the components (A) to (C) and the other components as required may be blended and manufactured by mixing using a known stirring device and the like.

The pH of the hair cosmetic of the present invention is not particularly limited as long as it is within the range generally applicable for use as a hair cosmetic, but from the viewpoint of suppressing damage to the hair and the scalp and facilitating to exhibit the effects of the present invention, the pH of the hair cosmetic is controlled to be preferably 3.0 or more and 8.0 or less, more preferably 3.2 or more and 7.0 or less, still more preferably 3.3 or more and 6.0 or less.

### [Hair treatment method]

The hair treatment method of the present invention contains a step of applying the above hair cosmetic of the present invention to the hair.

As the methods for applying the hair cosmetic to the hair, for example, applying, spraying or casting to the hair, immersing the hair into the hair cosmetic, and the like, are given. The hair to be applied may be dry or wet, preferably in the wet state by being washed after being combed with a brush, comb and the like. The application of the hair cosmetic may be applying or rubbing by hand, or performing by using a brush, comb, and the like. Moreover, the hair to which the hair cosmetic is applied may be the entire hair or part of the hair.

The amount of the hair cosmetic applied to the hair is as a bath ratio with respect to the weight of the hair to be applied (mass of the hair cosmetic/mass of the hair to be applied), preferably 0.03 or more, more preferably 0.1 or more, still more preferably 0.2 or more; preferably 20 or less, more preferably 15 or less, still more preferably 12 or less.

After the above step, it is preferable to perform a step of washing the hair to which the hair cosmetic was applied. In this case, it is preferable to set a leaving time after application of the hair cosmetic before washing with water so that the hair cosmetic can be sufficiently absorbed into the hair. The leaving time is preferably 15 seconds or longer, more preferably 30 seconds or longer, still more preferably 1 minute or longer; and is preferably 60 minutes or shorter, more preferably 30 minutes or shorter, still more preferably 20 minutes or shorter. It is also preferable to cover the hair to which the hair cosmetic was applied with a plastic film, a cap, and the like in order to inhibit the evaporation of water during the leaving.

The washing with water is sufficient as long as it can remove the excess hair cosmetics from the surface of the hair, and rinsing is preferable. The washing time is preferably 5 seconds or longer and 3 minutes or shorter. The temperature of the water may be such that it does not burden the body, preferably 15 °C or higher and 50 °C or lower, more preferably 25 °C or higher and 45 °C or lower.

The hair after the washing with water, is dried by a natural drying by towel drying and the like, or by a forced drying by using a dryer and the like.

By treating the hair, especially strong fizzy hair, using the hair cosmetic of the present invention as described above, it is possible to inhibit the shrinkage of the entire hair and impart the appropriate elastic texture.

In relation to the aforementioned embodiments, the preferable embodiments of the hair cosmetic of the present invention will be further disclosed as follows.

<1> A hair cosmetic containing the following components (A) to (C):
   (A) one or more carboxylic acids selected from the group consisting of a hydroxycarboxylic acid and a polyvalent carboxylic acid having no hydroxy group, or salts thereof;
   (B) an aromatic sulfonic acid or a salt thereof; and
   (C) a diethylene glycol monoalkyl ether,
   in which, a content of the component (A) is 0.8% by mass or more and 10.0% by mass or less.
<2> The hair cosmetic according to the item <1>, in which the hydroxycarboxylic acid of the component (A) is preferably one or more selected from hydroxymonocarboxylic acid having 2 to 5 carbon atoms, hydroxydicarboxylic acid having 3 to 8 carbon atoms, and hydroxytricarboxylic acid having 3 to 8 carbon atoms, more preferably one or more selected from hydroxydicarboxylic acid having 3 to 8 carbon atoms and hydroxytricarboxylic acid having 3 to 8 carbon atoms.
<3> The hair cosmetic according to the item <1> or <2>, in which the polyvalent carboxylic acid having no hydroxy group of the component (A), is preferably dicarboxylic acid having 3 to 8 carbon atoms and having no hydroxy group.
<4> The hair cosmetic according to any of the items <1> to <3>, in which the carboxylic acid of the component (A) is preferably one or more selected from malic acid, citric acid, succinic acid and lactic acid, more preferably one or more selected from malic acid and citric acid, still more preferably malic acid.
<5> The hair cosmetic according to any of the items <1> to <4>, in which the component (A) is preferably one or more selected from malic acid, citric acid, succinic acid and lactic acid, and salts thereof; more preferably one or more selected from malic acid, citric acid, and salts thereof; still more preferably malic acid or a salt thereof; even still more preferably one or more selected from malic acid, citric acid, succinic acid and lactic acid; even still more preferably one or more selected from malic acid and citric acid; even still more preferably malic acid.
<6> The hair cosmetic according to any of the items <1> to <5 >, in which the content of the one or more carboxylic acids selected from hydroxydicarboxylic acid having 3 to 8 carbon atoms and hydroxytricarboxylic acids having 3 to 8 carbon atoms, or the salt thereof in the component (A) is preferably 50% by mass or more, more preferably 70% by mass or more, still more preferably 90% by mass or more.
<7> The hair cosmetic according to any of the items <1> to <6 >, in which the content of the one or more carboxylic acids selected from malic acid and citric acid or the salts thereof in the component (A) is preferably 50% by mass or more, more preferably 70% by mass or more, still more preferably 90% by mass or more.
<8> The hair cosmetic according to any of the items <1> to <7 >, in which the aromatic sulfonic acid of the component (B) is preferably one or more selected from naphthalenesulfonic acids and benzenesulfonic acids; more preferably one or more selected from naphthalenesulfonic acid, xylenesulfonic acid, toluenesulfonic acid and cumenesulfonic acid; still more preferably one or more selected from naphthalenesulfonic acid and xylenesulfonic acid; even still more preferably naphthalenesulfonic acid.
<9> The hair cosmetic according to any of the items <1> to <8 >, in which the component (B) is preferably, one or more selected from naphthalenesulfonic acid, xylenesulfonic acid, toluenesulfonic acid, cumenesulfonic acid, and salts thereof; more preferably one or more selected from naphthalenesulfonic acid, xylenesulfonic acid, and salts thereof; still more preferably one or more selected from naphthalenesulfonic acid and a salt thereof; even still more preferably one or more selected from sodium 2-naphthalenesulfonate, sodium xylenesulfonate, sodium p-toluenesulfonate and sodium p-cumenesulfonate; even still more preferably one or more selected from sodium 2-naphthalenesulfonate, sodium xylenesulfonate and sodium p-toluenesulfonate; even still more preferably one or more selected from sodium 2-naphthalenesulfonate and sodium xylenesulfonate; even still more preferably sodium 2-naphthalenesulfonate.
<10> The hair cosmetic according to any of the items <1> to <9 >, in which the terminal alkyl group of the diethylene glycol monoalkyl ether of the component (C) is preferably an alkyl group having 2 to 6 carbon atoms, more preferably alkyl group having 2 to 4 carbon atoms.
<11> The hair cosmetic according to any of the items <1> to <10 >, in which the component (C) is preferably one or more selected from diethylene glycol monoethyl ether and diethylene glycol monobutyl ether, more preferably diethylene glycol monoethyl ether.
<12> The hair cosmetic according to any of the items <1> to <11 >, in which the content of the component (A) is preferably 1.0% by mass or more, more preferably 2.5% by mass or more; preferably 8.0% by mass or less, more preferably 7.0% by mass or less.
<13> The hair cosmetic according to any of the items <1> to <12 >, in which the content of the component (B) is, from the viewpoint of imparting the appropriate elastic texture to the hair, preferably 0.5% by mass or more, more preferably 1.0% by mass or more, still more preferably 1.5% by mass or more; preferably 10.0% by mass or less, more preferably 8.0% by mass or less, still more preferably 6.0% by mass or less.
<14> The hair cosmetic according to any of the items <1> to <13 >, in which the content of the component (C) is preferably 0.1% by mass or more, more preferably 0.2% by mass or more, still more preferably 0.5% by mass or more; preferably 25.0% by mass or less, more preferably 20.0% by mass or less, still more preferably 15.0% by mass or less.
<15> The hair cosmetic according to any of the items <1> to <14>, in which the mass ratio [(B)/{(A)+(C)}] of the content of the component (B) with respect to the total content of the component (A) and the component(C) is preferably 0.10 or more, more preferably 0.15 or more, still more preferably 0.18 or more; preferably 3.00 or less, more preferably 2.00 or less, still more preferably 1.00 or less.
<16> The hair cosmetic according to any of the items <1> to <15>, in which the mass ratio [(A)/{(A)+(B)}] of the content of the component (A) with respect to the total content of the component (A) and the component(B) is preferably 0.25 or more, more preferably 0.28 or more, still more preferably 0.30 or more; preferably 0.85 or less, more preferably 0.80 or less, still more preferably 0.75 or less.
<17> The hair cosmetic according to any of the items <1> to <16>, which is preferably hair shampoo, hair rinse, hair conditioner, hair treatment, hair pack or hair styling agent, more preferably hair rinse, hair conditioner, hair treatment, hair pack or hair styling agent.
<18> The hair cosmetic according to any of the items <1> to <17>, which is preferably a shrinkage inhibitor.
<19> Use of the hair cosmetic according to any one of the items <1> to <18> which is for shrinkage inhibition.
<20> A shrinkage inhibition method for hair in which the hair cosmetic according to any one of the items <1> to <18> is applied.
<21> A hair treatment method containing:
   a step of applying the hair cosmetic according to any one of the items <1> to <18> to the hair; and
   a step of washing the hair in which the hair cosmetic is applied.

### Examples

### [Examples 1 to 10, Comparative Examples 1 to 5]

The hair cosmetic having the compositions of the Examples and the Comparative Examples shown in Table 1 below were prepared, and the hair bundles for the evaluation were used to evaluate the effects of these hair cosmetics in inhibiting the shrinkage of the hair and imparting the appropriate elastic texture. The evaluation results are shown in Table 1.

### <Hair bundles for evaluation>

A curly hair (obtained from Kerling) of Caucasian with no chemical treatment history was placed in a line approximately 5 mm wide and fixed at the top end to form a hair bundle with a length of approximately 23 cm and a mass of 0.3 g when pulled and straightened. The hair bundle was washed with the plain shampoo composed of the composition shown below, and combed sufficiently with a comb (pins: 1 mm diameter, 40 pins, 2 mm apart; hereinafter the same) while being rinsed with tap water for 20 seconds. This hair bundle of a wet state was used as the hair bundle for the evaluation.

| (Composition of Plain Shampoo) | | |
|---|---|---|
| | | (% by mass) |
| | Sodium polyoxyethylene(2.5) lauryl ether sulfate | 15.5 |
| | Lauric acid diethanolamide | 2.3 |
| | Disodium edetate | 0.15 |
| | Sodium benzoate | 0.5 |
| | Sodium chloride | 0.8 |
| | Phosphoric acid | Moderate (pH adjustment) |
| | Fragrance | Trace amount (Less than 0.5) |
| | Methylparaben | Trace amount (Less than 0.5) |
| | Water | Balance |
| (pH 7.0) | | Total 100.0 |

### <Treatment of Hair bundles>

After 1 g of the hair cosmetic of each Examples and each Comparative examples was blended into the above hair bundle for the evaluation of the wet state, the entire hair bundle was covered with a plastic wrap film to be sealed, and left to stand at room temperature (20 °C) for 15 minutes. The hair bundle was then combed by the comb sufficiently while being rinsed with tap water for 20 seconds.

### <Shrinkage inhibition evaluations

For each of the untreated hair bundle for the evaluation and the treated hair bundle for the evaluation, the entire hair bundle was immersed in water in a beaker. The top end of the hair bundle was plucked, a comb was inserted under it, and the plucked top end was twisted clockwise while the comb was passed through the hair bundle and pulled up above the water surface to form the entire hair bundle into a spiral shape (see Fig. 1(a)). The top end of the hair bundle was fixed and hung, and the hair bundle was left to the natural drying at a temperature of 20 °C and a relative humidity of 40% for 90 minutes. At 90 minutes after the start of the natural drying, the length of the hair bundle was measured in its natural state without pulling (see Fig. 1(b) and 1(c)). The ratio (L₁/L₀) of the length (L₁) of the treated hair bundle at 90 minutes after the start of the natural drying to the length (L₀) of the untreated hair bundle for the evaluation at 90 minutes after the start of natural drying was calculated, and this value was used as the shrinkage improvement ratio. The measurements were performed to the three hair bundles for each hair cosmetic and the average value of the shrinkage improvement ratio for the three hair bundles is shown in Table 1.

When the shrinkage improvement ratio is greater than 1, it can be said that the shrinkage is inhibited, and it shows that the higher the value, the better the shrinkage inhibiting effect. The shrinkage inhibition ratio is preferably greater than 1.05, more preferably 1.08 or greater, still more preferably 1.10 or greater.

### <Evaluation of elasticity>

The elasticity of the hair bundles after the treatment was evaluated by five panelists using the tactile sensory evaluation. The evaluation was based on the following evaluation criteria. The average score of the five panelists is shown in Table 1 as the evaluation result of the elasticity.

When the evaluation score of the elasticity is 2.0 or greater and 4.0 or smaller, it can be said to have an appropriate elastic texture. The evaluation score of the elasticity is preferably 3.0 or greater and 4.0 or smaller.

### (Evaluation criteria)

1: No elasticity at all
2: Slightly elastic, but soft
3: Elastic
4: Elastic, but slightly hard
5: Hard

**[Table 1]**

| | | | Example | | | | | | | | | | Comparative Example | | | | |
|---|---|---|---|---|---|---|---|---|---|---|---|---|---|---|---|---|---|
| | | | 1 | 2 | 3 | 4 | 5 | 6 | 7 | 8 | 9 | 10 | 1 | 2 | 3 | 4 | 5 |
| Composition (% by mass) | | | | | | | | | | | | | | | | | |
| | (A) | Malic acid | 5.0 | 2.0 | 1.0 | 5.0 | 2.0 | 1.0 | 5.0 | - | 5.0 | 2.0 | - | - | 0.5 | 1.0 | 1.0 |
| | | Citric acid | - | - | - | - | - | - | - | 2.0 | - | - | - | 0.5 | - | - | - |
| | (A)' | Pyrrolidone carboxylic acid | - | - | - | - | - | - | - | - | - | - | 2.0 | - | - | - | - |
| | (B) | Sodium 2-naphthalene sulfonate | 4.0 | 2.5 | 2.0 | 2.0 | 2.0 | 2.0 | 2.0 | 2.0 | - | 4.0 | 2.5 | 2.0 | 2.0 | - | 4.0 |
| | | Sodium xylene sulfonate | - | - | - | - | - | - | - | - | 4.0 | - | - | - | - | - | - |
| | (C) | Diethylene glycol monoethyl ether | 10.0 | 5.0 | 5.0 | 2.0 | 10.0 | 10.0 | 1.0 | 5.0 | 10.0 | 2.0 | 5.0 | 5.0 | 5.0 | 5.0 | - |
| | Ethanol (Purity 95 vol%) | | 5.0 | 5.0 | 5.0 | 5.0 | 5.0 | 5.0 | 5.0 | 5.0 | 5.0 | 5.0 | 5.0 | 5.0 | 5.0 | 5.0 | 5.0 |
| | Benzyl alcohol | | 8.0 | 8.0 | 8.0 | 8.0 | 8.0 | 8.0 | 8.0 | 8.0 | 8.0 | 8.0 | 8.0 | 8.0 | 8.0 | 8.0 | 8.0 |
| | Dipropylene glycol | | 10.0 | 10.0 | 10.0 | 10.0 | 10.0 | 10.0 | 10.0 | 10.0 | 10.0 | 10.0 | 10.0 | 10.0 | 10.0 | 10.0 | 10.0 |
| | Sodium hydroxide (48% by mass aqueous solution) | | Moderate | Moderate | Moderate | Moderate | Moder- ate | Moderate | Moderate | Moderate | Moderate | Moderate | Moderate | Moderate | Moderate | Moderate | Moderate |
| | Purified Water | | Balance | Balance | Balance | Balance | Balance | Balance | Balance | Balance | Balance | Balance | Balance | Balance | Balance | Balance | Balance |
| | Total | | 100.0 | 100.0 | 100.0 | 100.0 | 100.0 | 100.0 | 100.0 | 100.0 | 100.0 | 100.0 | 100.0 | 100.0 | 100.0 | 100.0 | 100.0 |
| pH | | | 3.5 | 3.5 | 3.5 | 3.5 | 3.5 | 3.5 | 3.5 | 3.5 | 3.5 | 3.5 | 3.5 | 3.5 | 3.5 | 3.5 | 3.5 |
| Mass ratio of Components (B)/{(A) + (C)} | | | 0.27 | 0.36 | 0.33 | 0.29 | 0.17 | 0.18 | 0.33 | 0.29 | 0.27 | 1.00 | - | 0.36 | 0.36 | 0 | 4.00 |
| Mass ratio of Components (A)/{(A) + (B)} | | | 0.56 | 0.44 | 0.33 | 0.71 | 0.50 | 0.33 | 0.71 | 0.50 | 0.56 | 0.33 | 0 | 0.20 | 0.20 | 1.00 | 0.20 |
| Eval- uation | | Shrinkage improvement ratio | 1.16 | 1.14 | 1.15 | 1.09 | 1.13 | 1.08 | 1.12 | 1.10 | 1.08 | 1.15 | 1.04 | 1.05 | 0.99 | 1.17 | 1.05 |
| | | Elasticity | 3.5 | 4.0 | 4.0 | 3.5 | 2.5 | 3.5 | 3.5 | 3.0 | 3.0 | 4.0 | 4.0 | 5.0 | 5.0 | 1.0 | 5.0 |

Note that sodium hydroxide (48% by mass aqueous solution) in Table 1 was used as a pH adjuster for the hair cosmetics. The pH was measured at 25 °C with a pH meter ("Desktop pH meter F-21" manufactured by Horiba, Ltd.).

From the evaluation results in Table 1, it can be seen that the hair cosmetic of the present example can inhibit the shrinkage of the hair during drying after washing and can provide the appropriate elastic texture.

Hereinafter, the examples of the compositions of the other formulation examples of the hair cosmetic of the present invention are shown. In addition, the effect of inhibiting the shrinkage of the hair and imparting the appropriate elastic texture to the hair by these hair cosmetics was evaluated for Examples 13 and 15, using the hair bundles for the evaluation. The evaluation results are shown in Table 2.

### [Example 11]

| (Composition) | | [Unit: % by mass] |
|---|---|---|
| Component (A): | Succinic acid | 5.0 |
| Component (B): | Sodium 2-naphthalenesulfonate | 4.0 |
| Component (C): | Diethylene glycol monoethyl ether | 10.0 |
| Ethanol (purity 95 vol%) | | 5.0 |
| Benzyl alcohol | | 8.0 |
| Dipropylene glycol | | 10.0 |
| Sodium hydroxide (48% by mass aqueous solution) | | Moderate (pH adjustment) |
| Purified water | | Balance |
| (pH 3.5) | | Total 100.0 |

| | | |
|---|---|---|
| (Mass ratio of components (B)/{(A) + (C)} = 0.27) (Mass ratio of components (A)/{(A) + (B)} = 0.56) | | |

### [Example 12]

| (Composition) | | [Unit: % by mass] |
|---|---|---|
| Component (A): | Lactic acid | 5.0 |
| Component (B): | Sodium 2-naphthalenesulfonate | 4.0 |
| Component (C): | Diethylene glycol monoethyl ether | 10.0 |
| Ethanol (purity 95 vol%) | | 5.0 |
| Benzyl alcohol | | 8.0 |
| Dipropylene glycol | | 10.0 |
| Sodium hydroxide (48% by mass aqueous solution) | | Moderate (pH adjustment) |
| Purified water | | Balance |
| (pH 3.5) | | Total 100.0 |

| | | |
|---|---|---|
| Mass ratio of components (B)/{(A) + (C)} = 0.27 (Mass ratio of components (A)/{(A) + (B)} = 0.56) | | |

### [Example 13]

| (Composition) | | [Unit: % by mass] |
|---|---|---|
| Component (A): | Malic acid | 5.0 |
| Component (B): | Sodium p-toluenesulfonate | 4.0 |
| Component (C): | Diethylene glycol monoethyl ether | 10.0 |
| Ethanol (purity 95 vol%) | | 5.0 |
| Benzyl alcohol | | 8.0 |
| Dipropylene glycol | | 10.0 |
| Sodium hydroxide (48% by mass aqueous solution) | | Moderate (pH adjustment) |
| Purified water | | Balance |
| (pH 3.5) | | Total 100.0 |

| | | |
|---|---|---|
| (Mass ratio of components (B)/{(A) + (C)} = 0.27) (Mass ratio of components (A)/{(A) + (B)} = 0.56) | | |

### [Example 14]

| (Composition) | | [Unit: % by mass] |
|---|---|---|
| Component (A): | Malic acid | 5.0 |
| Component (B): | Sodium p-cumenesulfonate | 4.0 |
| Component (C): | Diethylene glycol monoethyl ether | 10.0 |
| Ethanol (purity 95 vol%) | | 5.0 |
| Benzyl alcohol | | 8.0 |
| Dipropylene glycol | | 10.0 |
| Sodium hydroxide (48% by mass aqueous solution) | | Moderate (pH adjustment) |
| Purified water | | Balance |
| (pH 3.5) | | Total 100.0 |

| | | |
|---|---|---|
| (Mass ratio of components (B)/{(A) + (C)} = 0.27) (Mass ratio of components (A)/{(A) + (B)} = 0.56) | | |

### [Example 15]

| (Composition) | | [Unit: % by mass] |
|---|---|---|
| Component (A): | Malic acid | 5.0 |
| Component (B): | Sodium 2-naphthalenesulfonate | 4.0 |
| Component (C): | Diethylene glycol monobutyl ether | 10.0 |
| Ethanol (Purity 95 vol%) | | 5.0 |
| Benzyl alcohol | | 8.0 |
| Dipropylene glycol | | 10.0 |
| Sodium hydroxide (48% by mass aqueous solution) | | Moderate (pH adjustment) |
| Purified water | | Balance |
| (pH 3.5) | | Total 100.0 |

| | | |
|---|---|---|
| Mass ratio of components (B)/{(A) + (C)} = 0.27 (Mass ratio of components (A)/{(A) + (B)} = 0.56) | | |

**[Table2]**

| | | | Example | |
|---|---|---|---|---|
| | | | 13 | 15 |
| Composition (% by mass) | | | | |
| | (A) | Malic acid | 5.0 | 5.0 |
| | (B) | Sodium 2-naphthalenesulfonate | - | 4.0 |
| | | p-toluenesulfonic acid | 4.0 | - |
| | (C) | Diethylene glycol monoethyl ether | 10.0 | - |
| | | Diethylene glycol monobutyl ether | - | 10.0 |
| | Ethanol (Purity 95 vol%) | | 5.0 | 5.0 |
| | Benzyl alcohol | | 8.0 | 8.0 |
| | Dipropylene glycol | | 10.0 | 10.0 |
| | Sodium hydroxide (48% by mass aqueous solution) | | Moderate | Moderate |
| | Purified Water | | Balance | Balance |
| | Total | | 100.0 | 100.0 |
| pH | | | 3.5 | 3.5 |
| Mass ratio of Components (B)/{(A) + (C)} | | | 0.27 | 0.28 |
| Mass ratio of Components (A)/{(A) + (B)} | | | 0.56 | 0.56 |
| Evaluation | | Shrinkage improvement ratio | 1.07 | 1.17 |
| | | Elasticity | 3.0 | 3.5 |

When the hair bundles for the evaluation were treated in the same manner as in Example 1 using the hair cosmetics of Examples 11 to 15, the shrinkage of the hair during drying after washing was inhibited, and the appropriate elastic texture was obtained. In particular, from the results in Table 2, it can be seen that the hair cosmetics of Examples 13 and 15 can inhibit the shrinkage of the hair during drying after washing, and can provide the appropriate elastic texture.

### Industrial Applicability

The hair cosmetic of the present invention can inhibit the shrinkage of the hair during drying after washing, can impart the appropriate elastic texture, and in particular, can be suitably applied to the efficient styling of the strong fizzy hair.

## Claims

1. A hair cosmetic comprising the following components (A) to (C):
(A) one or more carboxylic acids selected from the group consisting of a hydroxycarboxylic acid and a polyvalent carboxylic acid having no hydroxy group, or salts thereof;
(B) an aromatic sulfonic acid or a salt thereof; and
(C) a diethylene glycol monoalkyl ether, wherein,
a content of the component (A) is 0.8% by mass or more and 10.0% by mass or less.

2. The hair cosmetic according to claim 1, wherein the carboxylic acid of the component (A) is one or more selected from malic acid, citric acid, succinic acid and lactic acid.

3. The hair cosmetic according to claim 1 or 2, wherein the aromatic sulfonic acid of the component (B) is one or more selected from naphthalenesulfonic acids and benzenesulfonic acids.

4. The hair cosmetic according to any one of claims 1 to 3, wherein the component (B) is one or more selected from sodium 2-naphthalenesulfonate, sodium xylenesulfonate, sodium p-toluenesulfonate and sodium p-cumenesulfonate.

5. The hair cosmetic according to any one of claims 1 to 4, wherein the component (C) is one or more selected from diethylene glycol monoethyl ether and diethylene glycol monobutyl ether.

6. The hair cosmetic according to any one of claims 1 to 5, wherein a mass ratio [(B)/{(A)+(C)}] of the content of the component (B) with respect to the total content of the component (A) and the component (C) is 0.10 or more and 3.00 or less.

7. The hair cosmetic according to any one of claims 1 to 6, wherein a mass ratio [(A)/{(A)+(B)}] of the content of the component (A) with respect to the total content of the component (A) and the component (B) is 0.25 or more and 0.85 or less.

8. The hair cosmetic according to any one of claims 1 to 7, wherein a content of the component (B) is 0.5% by mass or more and 10.0% by mass or less.

9. The hair cosmetic according to any one of claims 1 to 8, wherein a content of the component (C) is 0.1% by mass or more and 25.0% by mass or less.

10. The hair cosmetic according to any one of claims 1 to 9, which is a shrinkage inhibitor.

11. Use of the hair cosmetic according to any one of claims 1 to 10, which is for shrinkage inhibition.

12. A hair treatment method comprising:
a step of applying the hair cosmetic according to any one of claims 1 to 10 to a hair; and
a step of washing the hair in which the hair cosmetic is applied.
